# EUROPEAN PATENT APPLICATION

(11) **EP 2 692 330 A2**
(43) Date of publication of application: **05.02.2014**
(21) Application number: 12726724.3
(22) Date of filing: 30.03.2012
(51) Int. Cl.: A61K 8/19, A61K 8/92, A61Q 19/10

(54) **COSMETIC CLEANSING COMPOSITION COMPRISING VEGETABLE OILS**

(30) Priority: 31.03.2011 BR PI1101133
(71) Applicant: Natura Cosmeticos S.A., 06882-700 ltapecerica da Serra - SP (BR)
(72) Inventor: BRASILINO DE CARVALHO, André Luís, Sâo Bernardo do Campo, SP (BR); PEREIRA LIMA, Rodrigo, Jundiaí, SP (BR); RUBENS MIGUEL, Reinaldo, Jundiaí, Sp (BR); DE OLIVEIRA MARIN CHICOL, Tadeu, 07760-000 Cajamar SP (BR)
(74) Representative: Hewson, Timothy John
(86) International application number: PCT/BR2012/000088
(87) International publication number: WO 2012/129627

(57) **Abstract**

The present invention relates to a cosmetic composition, particularly indicated for cleaning the skin, which comprises a plant oil portion comprising:
- at least 70% of fatty chain with 18 carbon atoms, C18, such chain may be either saturated or unsaturated;
- up to 20% of fatty chain with 16 carbon atoms, C16, such chain may be either saturated or unsaturated;
- at least one saponifying agent;

and an aqueous portion.

Further, the invention relates to a process for preparing the cosmetic composition and the use of this composition.

Such a cosmetic composition has acceptance and pleasantness that are superior to those of the prior-art products, as well as extremely superior formation of film.

## Description

### FIELD OF INVENTION

The present invention relates to cosmetics. More specifically, the present invention relates to cosmetic compositions, particularly those indicated for skin cleaning comprising vegetable (plant) oils, as well as to the process of manufacturing these compositions, besides the use thereof.

### BACKGROUND OF THE INVENTION

Toilet soap is a solid or liquid object, composed by amphiphilic molecules composed by metal salts, especially sodium hydroxide or potassium and fatty acids. Its amphiphilic nature imparts to it its characteristic properties, including the formation of foam and of emulsions suitable for washing.

Commercial soaps are manufactured through a saponification reaction from a mixture of fats, glycerol triester and fatty acids, usually called fatty acid triglycerides and a strong base. The usual soap molecules have a chain of 8 to 19 carbon atoms, together with a polar "head".

The soaps have various shapes, depending of their water contents, presence of fat or other impurities. When they are dry, they form brittle solids. When wet and soaked, they slide on solid surfaces, becoming soft and losing their dimensional stability, until finally they liquefy. These observations confirm their colloid nature, as well as their other forms of foam, gel, etc.

Fatty acid salts are not soluble in water and oil. Indeed, they are amphiphilic, that is, they are located at the interface of the aqueous and oily phases, being immiscible. In the absence of one of the phases, they form single molecular structures, called micelles.

Commercial soaps are mixtures of sodium or potassium salts of fatty acids. The length of the carbons chain and, especially, the presence of unsaturation, that is, a double bond inducing to a space conformation, rigidity or a specific mobility, affect the properties of the final product.

The principle of action of the carboxylates molecules (R-CO₂) of bases (Na⁺, K⁺) is due to their amphiphilicity: they represent a long carbon chain, which is not polar and, at their end, a carboxylate group. The first long part is non-polar, therefore, hydrophobic and lipophilic. It is the tail of fatty acids that mixes easily with fat. The second part ("head") is polar and hydrophilic, and therefore minimizes its energy because it is in contact with an aqueous solution.

In the presence of an ionic solvatation mist in the double layer above the hydrophilic surface, the micelle is stabilized.

The detergent properties of soap water are explained as follows: soaps, due to their lipophilic tail, binds to the fatty dirt or oil stain and remove it from the fabric or support by involving it in colloids or spherical fats that detach and join myriads of micelles.

During the bath, the soap dissolves the fat that constitutes the hydroiipidic film that covers the skin. The fat is drawn into the water with the dirt which it contains. The disadvantage is that the hydrolipidic film serves to protect the skin and retain its water. Washing with products containing surfactants, such as bath gels, weaken the skin until the hydrolipidic film is reconstituted, after several hours.

The soap is basic. Since its pH is close to 10, in a concentrated and hot solution, like bath water, it interferes with the acidity of the skin, which has a pH close to 5.

Handcrafted or industrial soap is the product of a chemical reaction called fat saponification. This irreversible and slow transformation is one of the oldest chemical reactions known and controlled by mankind. It is a simple alkaline hydrolysis, during which a mixture of fats - animal fats or plant oils - is hydrolyzed in an alkaline medium with a strong potassa base or potassium hydroxide, KOH, soda or sodium hydroxide at a temperature ranging from 80°C to 100°C.

The raw materials for manufacturing soaps are fat and soda, possible potassa. A well finished soap does not contain soda or oil. Soaps are essentially different sodium carboxylates, soap molecules. They also contain water and various additives.

In their simplest form, soap is a totally biodegradable detergent product. The additives, however, may pollute the environment.

Most researches of the cosmetic industries that manufacture soaps aim at improving the germicidal or antiseptic properties of the soaps manufactured. However, at present there is a need to offer the consumer soaps that, keeping their germicidal, antiseptic, nutritive and/or moisturizing properties intact, provide an aspect that is appealing to the senses.

Document US 6,376,438 discloses cleaning agents in the form of a hydrated liquid paste or similar to a cream without organic solvents, being especially a product for cleaning hands. These agents comprise from 10 to 30% of at least one plant oil, from 10 to 30% of ethoxylated fatty alcohol, at least one fatty alcohol ether sulfate and a salt of sulfonated fatty acid, water and an abrasive.

Document US 7,749,523 discloses an exfoliating cosmetic composition for use in cleaning and conditioning the skin of hand, face, joints, which does not leave a heavy or greased when the composition is applied to the skin and then rinsed. This composition comprises high a concentration of oils in the function of emollients.

Therefore, no prior-art teaching discloses a cosmetic composition indicated particularly for cleaning the skin, comprising concentration of plant oils with a balance of average fatty chains ranging from C16 to C18, exhibiting paste consistency, providing efficiency in cleaning and softening the skin.

### BRIEF DESCRIPTION OF THE INVENTION

The present invention relates to a cosmetic composition, particularly indicated for skin cleaning, which comprises:
- an oily portion of plant oil comprising:
- at least 70% of fatty chain with 18 carbon atoms, C18, such chain may be either saturated or unsaturated;
- up to 20% of fatty chain with 16 carbon atoms, C16, such chain may be saturated or unsaturated;
- at least one saponifying agent; and
- an aqueous portion.

This cosmetic composition exhibits consistency, texture and physical properties other than those of cosmetic compositions known on the market for the same purposes. Especially, one points out the consistency of this composition, which is intermediate between products in the form of bars and liquid products, being classified herein as a paste.

Further, the present invention relates to the process of preparing this cosmetic composition, which comprises the following steps:
a) preparing an oily portion comprising at least one plant oil that comprises:
   - at least 70% of fatty chain with 18 carbon atoms, C18, such chain may be either saturated or unsaturated;
   - up to 20% of fatty chain with 16 carbon atoms, C16, such chain may be either saturated or unsaturated;
b) heating the oily portion of step a) up to a temperature ranging from about 90°C to 95°C;
c) after heating, adding slowly at least one saponifying agent, under constant stirring and high rotation;
d) adding slowly an aqueous portion;
e) checking the reduction of steam formed with the reaction.

And further the invention relates to the use of the cosmetic composition objectified above for cleaning and hygiene of the skin.

### BIEF DESCRIPTION OF THE FIGURES

The present invention will now be described in greater detail with reference to the results of tests made by using the cosmetic composition of the present invention:
figure 1: illustration of the images of optical microscopy representative of the study groups, showing comparison between control and group that used the cosmetic composition of the present invention; and
figure 2: illustration of the characterization of films on Vitro-Skin^{™} by optical microscopy, showing comparison between control and group that used the cosmetic composition of the present invention.

### DETAILED DESCRIPTION OF THE INVENTION

The present invention relates to a cosmetic composition, particularly indicated for cleaning the skin, which comprises:
- an oily portion of vegetable origin, comprising:
- at least 70% of fatty chain with 18 carbon atoms, C18, such chain may be either saturated or unsaturated;
- up to 20% of fatty chain with 16 carbon atoms, C16, such chain may be either saturated or unsaturated;
- at least one saponifying agent; and
- an aqueous portion.

Preferably, the cosmetic composition object of the present invention comprises:
- an oily portion of plant origin comprising plant oil that comprises
- at least 70% of fatty chain with 18 carbon atoms, C18, such chain may be either saturated or unsaturated;
- up to 20% of fatty chain with 16 carbon atoms, C16, such chain may be either saturated or unsaturated;
- in an amount ranging from 50% to 70% by mass;
- a saponifying agent in an amount ranging from 15% to 25% by mass; and
- water,

All the amounts are based on the total mass of the cosmetic composition.

The present invention provides, in one of its preferred embodiments, a toilet soap in paste with a uniform specific consistency, with the property of forming a film that is significantly superior to the control (water), using a simple, effective and cost-saving process.

In one first preferred embodiment, the object of the present invention comprises:
- an oily portion of plant origin, comprising passiflora oil in an amount ranging from 25% to 70% by mass;
- a saponifying agent, which is sodium hydroxide in an amount ranging from 15 to 25% by mass; and
- water;

All the amounts are based on the total mass of the cosmetic composition.

In a second preferred embodiment, the object of the present invention comprises:
- an oily portion of plant origin comprising passiflora oil in an amount ranging from 25% to 70% by mass and palm olein in an amount ranging from 0% to 25% by mass:
- a saponifying agent, which is sodium hydroxide in an amount ranging from 15% to 25% by mass; an
- water;

All the amounts are based on the total mass of the cosmetic composition.

The cosmetic composition of the present invention has a range of advantages and characteristics that are desired in a cosmetic product for the skin, these advantages being achieved with the optimum combination between the components already described, some of which are cited below:
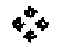 It provides efficient cleaning of the skin, making it smooth, silky, soft;
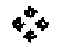 The cosmetic composition does not dry with passage of time;
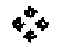 It maintains the ideal consistency as time goes by;
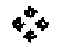 The cosmetic composition of the present invention brings a great innovation in the socio-environmental field: the incorporation of 20% to 50% of oils from the Brazilian Biodiversity in manufacturing of the vegetable masses.

This work has generated employments for over 830 associates of 10 new cooperatives/communities in the surroundings of the Saboaria Natura in the city of Benevides, PA, Brazil.

The composition of the present invention can be used for preparing toilet soaps in the form of paste.

Further, the composition of the present invention can be used, for instance, in beauty salons, esthetic activity centers, spas, among others.

### Plant Oil

Passiflora oil, mentioned as component of the first preferred embodiment of the present invention, enables one to manufacture a cosmetic composition that has consistency of paste and is creamy, being ideal for use with a sponge to clean the skin. Further, it provides the skin with a protective film that is six times as thick as that of ordinary soaps, without drying the skin and making it soft.

Palm olein, cited in the second preferred embodiment of the invention, may also be used in combination with other plant oil, as long as such a combination will obey the balance between C16 and C18 fatty chains.

Further, there are other plant oils that can be used in the constitution of the oily portion of the present invention. Preferred examples of plant oil indicated for preparing the cosmetic composition of the present invention are: maize, canola, sunflower, pea-nut, soybean.

The plant oils mentioned above can be used in isolation in the oily portion of the present invention or combined, as long as such a combination will be limited to:
- at least 70% of fatty chain with 18 carbon atoms, C18, such chain may be either saturated or unsaturated;
- up to 20% of fatty chain with 16 carbon atoms, C16, such chain may be either saturated or unsaturated.

Other plant oils may be added to the oily portion of the present invention, as long as they are combined with at least one of the plant oils cited above or others that are known from the literature and have, in their constitution, an adequate amount of fatty acids with middle chain of C16 and C18, and as long as such a mixture obeys the balance of fatty chain already explained.

In a preferred embodiment of the present invention, the cosmetic composition comprises an oily portion, which comprises passiflora oil combined with palm olein, wherein the mixture should comprise - at least 70% of fatty chain with 18 carbon atoms, C18, such chain may be either saturated or unsaturated;
- up to 20% of fatty chain with 16 carbon atoms, C16, such chain may be either saturated or unsaturated.

### Saponifying agent

Saponification is basically the interaction that takes place between a fatty acid existing in oils with a strong base under heating. The soap is a carboxylic acid salt and, since it has a long carbon chain in its molecular structure, it is capable of solubilizing in both polar and non-polar media.

For preparing the cosmetic composition of the present invention one uses a saponifying agent, a strong base being preferable.

Preferably, one adds, as a saponifying agent, sodium hydroxide in an amount ranging from 15% to 25% by mass.

### Antioxidant

In embodiments of the invention, one adds at least one antioxidant, vitamin E being preferred, in an amount ranging from 0% to 0.5% by mass, the amounts being based on the total mass of the composition.

Vitamin E performs a fundamental role in preserving the cell membranes by virtue of its action of capturing free radicals formed in the biochemical reactions in the skin or caused by exposure to sunlight. Thus, vitamin E interrupts the cascade effect of formation of more radicals, protecting the skin structures (colloidal gel, elastin and collagen) and minimizing the degradation thereof.

Studies carried out demonstrated that the antioxidant complex comprising licopen, vitamin E, acetate and coffee extract exhibit a synergistic effect, thus helping the natural defense system of the organism to fight more efficiently the free radicals formed from external aggressive elements, such as ultraviolet rays.

### Optional components

In order to impart to the cosmetic composition of the present invention some desirable characteristic that has not yet been achieved with the components already cited, one may add optional components that are compatible with its properties. Some of these components that may be added to the composition are described hereinafter.
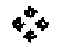 diphosphonates (bisphosphonates) may be present in amounts ranging from about 0.01 to about 0.20% by mass, and be selected from the group consisting of etidronic acid, Turpinal SL and a mixture thereof;
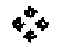 wetting agents (or hydrating agents or emollients) may be present in amounts ranging from about 1 to about 10.00% by mass, and may be selected from the group consisting of propyleneglycol, sucrose, sorbitol, glycerin, vaseline, mineral oil (s) and a mixture thereof;
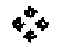 hardening agents may be present in amounts ranging from about 0.10% to about 0.50% by mass and may be selected from the group consisting of sodium chloride, sodic carboxymethylcellulose, dietanolamine of fatty acid (s) and a mixture thereof;
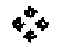 chelating agents may be present in amounts ranging from about 0.30% to about 0.70% by mass and may be selected from the group consisting of tetrasodic EDTA;
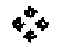 dyes (or pigments) may be present in amounts ranging from about 0.001 % to about 0.030% by mass;
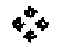 antioxidant agents may be present in amounts ranging from about 0.01 % to about 0.10% by mass and may be selected from the group consisting of BHT (buthylhydroxytoluene);
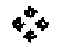 surfactants may be present in amounts ranging from about 0.10% to about 10.00% by mass and may be selected from the group consisting of decyl glucoside, lauryl sodium sulfate and a mixture thereof;
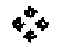 perfume (or essence) may be present in amounts ranging from about 0.01 % to about 2.00% by mass;
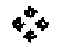 film former for bar soap, preferably lecithin, in an amount ranging from about 0.10% to 2.00% by mass;
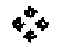 emulsifiers, preferably steareths, in an amount that varies according to the calculation of HLB of the formulation.
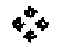 other cosmetically acceptable components.

All the amounts cited for the optional components are based on the total mass of the final formulation of a soap that contains the composition of the present invention. Preferably, the composition of the present invention should be present in amounts ranging from 60 to 95% by mass, based on the total mass of the final composition of a soap.

### Example of formulation of the soap in paste form of passiflora:

### Process for preparing the cosmetic composition of the present invention

The procedure for preparing the cosmetic composition of the present invention is superior to the usual procedures in that, starting from only 3 components and simple steps, it provides the cosmetic composition of the present invention that exhibits superior properties if compared with the products existing on the market.

The process of preparing the cosmetic composition object of the present invention comprises the following steps:
a) preparing an oily portion comprising at least one plant oil that comprises:
   - at least 70% of fatty chain with 18 carbon atoms, C18, such chain may be either saturated or unsaturated;
   - up to 20% of fatty chain with 16 carbon atoms, C16, such chain may be either saturated or unsaturated;
b) heating the oily portion of step a) up to a temperature from about 90°C to 95°C;
c) after heating, adding slowly at least one saponifying agent, under constant stirring and at high rotation;
d) adding slowly an aqueous portion;
e) checking the reduction of steam formed with the reaction.

Optionally, in step a), one adds an antioxidant agent in the oily portion.

Further, optional steps may be included in the process described above:
f) adding to the mixture of phase e) etidronic acid in an amount ranging from about 0.01 to about 0.20%;
g) adding to the mixture of phase f) polyalcohol, preferably sorbitol, in an amount ranging from 1 to 10%;
h) adding to the mixture of step g) at least one surfactant, so as to impart to the cosmetic composition greater foaming capability in an amount ranging from 0.10% to about 10.00%;
i) adding to the mixture of step h) a chelating agent, preferably EDTA in an amount ranging from 0.01 to about 0.20%;
j) adding to the mixture of step i) emulsifiers, preferably stearates, in an amount that varies depending on the calculation of HLB of the formulation.

All the amounts presented for the components of the optional steps are based on the total mass of the final formulation of a toilet soap containing the composition of the present invention. Preferably, the composition of the present invention should be present in the amounts ranging from 60 to 95% by mass, based on the total mass of the final formulation of a toilet soap.

The process of preparing the cosmetic composition of the present invention results then in a cosmetic composition intended for cleaning the skin, a toilet soap in paste with a specific consistency being preferably, so as to exhibit the property of forming a film.

In a first preferred embodiment, the process of preparing the cosmetic composition object of the present invention comprises the following steps:
a) preparing an oily portion comprising at least one plant oil comprising
   - at least 70% of fatty chain with 18 carbon atoms, C18, such chain may be either saturated or unsaturated;
   - up to 20% of fatty chain with 16 carbon atoms, C16, and such a chain may be either saturated or unsaturated,
      in an amount ranging from 50% to 70% by mass;
b) heating the oily portion of step a) up to a temperature of about 90°C to 95°C;
c) after the heating, adding slowly at least one saponifying agent, under constant stirring and at high rotation, in an amount ranging from 15% to 25% by mass;
d) adding slowly an aqueous portion;
e) checking the reduction of steam formed with the reaction;

Optionally, in step a) one adds an antioxidant agent in the oily portion.

In a preferred embodiment, the process of preparing the cosmetic composition object of the present invention comprises the following steps:
Initially, three different solutions are formed, namely: solution A (solubilized dyes), solution B (solubilized emulsifiers) and solution C (essence and solubilized antioxidants).

To solution B one adds the components of Phase 1 (palm olein, plant oil and oily vitamin E) under stirring and heating (preferably between 90 and 95°C). Then, one adds liquid caustic soda and water under stirring and heating. A chelating agent is added. Then, a polyalcohol with water is added, still under stirring. Finally, a surfactant and an emulsifier are added.

To this mixture one adds the phases A and C, together with a chelating agent and water.

Preferably, the present invention comprises the steps detailed below.

The first step of the process is weighting the raw material of the 12 phases. As preferred components of Phase 1, one employs palm olein PN4, filtered crude passiflora oils and oily vitamin E.

Then, one adds, in a boiler, demineralized water and dye, preferably tartarazin yellow. One stirs it until homogenization is completed.

In parallel, one adds, in a tank with heating, steareth 21 and steareth 2. One heats it under stirring up to 50°C.

In parallel, one adds, in a boiler, the essence, preferably passiflora essence, and BHT. One stirs it until homogenization is completed.

Then one adds to the tank the raw materials of Phase 1, in the following order: palm olein ON4, filtered crude passiflora oil and oily vitamin E. one stirs for 3 minutes with a scraper 60Hz and turned-on turbine. Then one starts the heating under stirring (90-95°C).

One adds slowly, through a pump, with a scraper 60Hz and turned-off turbine, the raw materials in the following order:
1. liquid caustic soda - pump velocity of 20 to 22Hz (average addition time: 15 minutes);
2. demineralized water - pump velocity of 38 - 40Hz (average addition time: 30 minutes).

After adding the demineralized water, one turns off the heating and turns on the turbine, keeping it on for 8 minutes. One turns off the turbine and keeps only the scraper on. One stirs for more 10 minutes.

One adds etidronic acid / turpinal, and stirs for 2 minutes with the scraper 40Hz. Then, one adds the tetrasodic EDTA in 40% solution and stirs for 2 minutes with a scraper 40Hz.

With the temperature below 90°C, one adds slowly, through a pump, with the scraper 40Hz, the raw materials in the following order:
1. Liquid sorbitol - pump velocity of 20 to 26 Hz (average addition time: 5 minutes);
2. demineralized water - pump velocity of 37 to 40Hz (average addition time: 16 minutes).

Then, one adds, in order, decyl polyglycose 2000 and lecithin. One initiates the cooling and adds the dying solution, under stirring for 5 minutes with a scraper 40Hz.

One adds etidronic acid/turpinal SL, under stirring for 3 minutes with a scraper 40Hz and steareth 21 and steareth 2 in water-bath.

Tetrasodic EDTA - 40% solution is added under stirring for 3 minutes with a scraper 40Hz.

One adds slowly, through a pump, with scraper 40Hz, demineralized water with pump velocity of 36 to 38Hz, in an average addition time of 20 minutes. One stirs for 5 minutes with a scraper 40Hz and, as soon as the temperature is below 52°C, one adds the essence with the BHT. One adds for 10 minutes with a scraper 40Hz.

Demineralized water is added slowly through a pump with a scraper 40Hz, with pump velocity of 36-38Hz, in an average addition time of 20 minutes. One stirs for 10 minutes with a scraper 40Hz.

Finally, a product with density of 0.7 to 0.85g/cm³ is obtained.

### TESTS CARRIED OUT

### Test 1

A quantitative study evaluating the performance of the cosmetic composition of the present invention was carried out. In total, 120 monadic evaluations (blind test) were made:
The profile of the respondents was determined as follows:
   - Women (100%)
   - With age between 18 and 45 years
   - 18-30 → 50%
   - 31-45 → 50%
   - Classes AB
   - Class A → 50%
   - Class B → 50%
   - Users of bar toilet soap in the bath, minimum of 7 days per week.

The result was positive, having acceptance and pleasantness that were superior to the products of the prior art used in the test. The main characteristics pointed out in the cosmetic composition of the present invention were: result felt on the skin, aroma and foaming, wherein the paste format showed greater differentiation, being superior to all the other products tested.

### Test 2: Evaluation of the formation of film on VitroSkin^{™} by optical microscopy.

The present study had the objective of evaluating the efficacy of the sample of product: paste toilet soap applied onto the artificial skin for the attribute of film formation.

The study was carried out in vitro, using Vitroskin^{™} (artificial skin) as a substrate, and the measures were made by the technique of optical microscopy.

The experimental procedure used can be summarized in the following steps:

### 1. Procedures

### 1.1. Preparation of Vitro-Skin^{™}

30 VitroSkin^{™} substrates measuring 3.0cm X 2.5cm were prepared, which were allocated to plastic slides for fixation, supplied by IMS-USA, providing a free area for product application of 5.0 cm². Prior to utilization, the substrates were maintained for 16 hours in a hydration chamber (IMS-USA) containing 15% (m/m) solution of glycerin in water.

### 1.2. Standard application of the products onto the substrate

Control group: 10 pL of distilled water are applied onto 3 previously hydrated substrates, massage was made by using an inox point with a 6.0 mm diameter for 30 seconds with circular movements, followed by drying in an oven at 35°C for 1 hour.

### Sample Group:

- Toilet soap in paste: 0.5 g of the product was applied onto 3 previously hydrated substrates, massage was made by using an inox point having 6.0 mm diameter for 30 seconds with circular movements, followed by rinsing with distilled water for 30 seconds and drying in an oven at 35°C for 1 hour.

### 1.3. Evaluation by optical microscopy

Optical microscopy was carried out by using the equipment Fluorescence Leica DMR300 Microscope, using cubic N2.1 filters. 3 regions were analyzed for each substrate, in a total of 9 regions per product.

### 2. Software for purchase and analysis of the data

Leica QWin (Leica, Germany);
Scion Image for Windows (Scion Corp. NIH, USA);
Microsoft^{™} Office Excel 2003 (Microsoft, USA);
GraphPad Prism 4.03 (GraphPad, California, USA).

### RESULT:

One used Vitro-Skin^{™} as substrate, supplied by IMS Inc (USA). According to the manufacturer, Vitro-Skin contains the protein and lipid components of the skin, mimicking the topography, pH, critical surface tension and ionic force of the human skin (http://www.ims-usa.com).

Figure 1 illustrates a few images of optical microscopy of the Vitro-Skin^{™} substrates, obtained after application of distilled water (Controlleft image) and after application of the analyzed sample (right figure).

The deposition of materials onto the rough surface of Vitro-Skin^{™} (R_{MS} = 80µm) alters the phase contrast which is observed in optical microscopy. The formation of clear or dark regions in the images of optical microscopy depends on the physicochemical characteristics of the depositing material. The substrates treated with the product exhibited formation of dark areas referring to the material deposited.

Through the analysis of images, the images obtained are binarized, and the region referring to the material deposited is identified as black pixels - figure 2 (film = green section; substrate = red section).

The images obtained were binarized by adjusting the 8-bits histogram to the range of 0-130, determining the percentage of black pixels (C). The higher the percent of black pixels, the larger the amount of material deposited onto the Vitro-Skin^{™} surface. The product analyzed had count of pixels of 25.7.

The results obtained for the product evaluated, considering the three treatments, were statistically evaluated with respect to the Control, applying the t-Student test, bimodal, non-paired, considering an interval of trust of 95%, to the date of count of black pixels, C, obtained for each study group. The product in question achieved a result of P=0.0005.

According to the results of the statistic analysis, the application of the product exhibited film formation that was extremely superior (P=0.0005) to the control (Vitro-Skin^{™}) treated with water alone).

According to the results of the study carried out, the product in question exhibited film formation that was extremely superior to the control (water). This indicated that this product exhibited film formation property when applied to the skin.

The illustrative examples presented hereinafter will serve to describe the present invention in a better manner. However, the formulations described refer merely to a few embodiments of the present invention and should not be taken as being limitative of the scope thereof. Further, the formulations exemplified were obtained by using the steps of the preparation process described before in the present specification.

### Example of Formulation 1

| **Component** | **Concentration (% by weight)** |
|---|---|
| Etidronic acid | 0.1000 |
| Tetrasodic EDTA | 0.5000 |
| Passiflora oil | 0.1000 |
| Sorbitol | 3.0000 |
| BHT | 0.0500 |
| Tartarazin yellow | 0.0025 |
| Mass for passiflora toilet soap in paste | 94.5475 |
| Passiflora S2 MOD II | 1.7000 |

### Example of Formulation 2

| **Component** | **Concentration (% by weight)** |
|---|---|
| Etidronic acid | 0.1000 |
| Demineralized water | 18.000 |
| Decyl glicoside | 2.0000 |
| Tetrasodic EDTA | 0.5000 |
| Passiflora oil | 3.0000 |
| Sorbitol | 5.0000 |
| Esteareth-2 | 1.6000 |
| Esteareth-21 | 0.6000 |
| BHT | 0.0500 |
| Lecithin | 1.0000 |
| Tartarazin yellow | 0.0025 |
| Mass for passiflora toilet soap in paste | 66.4475 |
| Passiflora S2 MOD II | 1.7000 |

### Example of Formulation 3

| **Component** | **Concentration (% by weight)** |
|---|---|
| Etidronic acid | 0.1000 |
| Demineralized water | 18.0000 |
| Decyl glycoside | 2.0000 |
| Tetrasodic EDTA | 0.5000 |
| Sorbitol | 5.0000 |
| Esteareth-2 | 1.6000 |
| Esteareth-21 | 0.6000 |
| BHT | 0.0500 |
| Lecithin | 1.0000 |
| Tartarazine yellow | 0.0025 |
| Mass for passiflora toilet soap in paste | 69.4475 |
| Passiflora S2 MOD II | 1.7000 |

### Example of Formulation 4

| **Component** | **Concentration (% by weight)** |
|---|---|
| Etidronic acid | 0.1000 |
| Demineralized water | 12.0000 |
| Tetrasodic EDTA | 0.5000 |
| Lauril ether sodium sulfate | 5.0000 |
| Passiflora oil | 0.5000 |
| Sorbitol | 10.0000 |
| BHT | 0.0500 |
| Tartarazine yellow | 0.0025 |
| Mass for passiflora toilet soap in paste | 70.1475 |
| Passiflora S2 MOD II | 1.7000 |

## Claims

1. A cosmetic composition, **characterized by** comprising a plant oil portion comprising:
- at least 70% of fatty chain with 18 carbon atoms, C18, such chain may be either saturated or unsaturated;
- up to 20% of fatty chain with 16 carbon atoms, C16, such chain may be either saturated or unsaturated;
- at least one saponifying agent;
and an aqueous portion.

2. A cosmetic composition according to claim 1, **characterized in that** the oily portion is present in an amount ranging from 50% to 70% by mass, based on the total mass of the composition.

3. A cosmetic composition according to claim 1 or 2, **characterized in that** the oil portion comprises at least one plant oil selected from passiflora oil, maize oil, canola oil, sunflower oil, soybean oil and mixtures thereof.

4. A cosmetic composition according to claim 3, **characterized in that** the oily portion comprises passiflora oil in an amount ranging from 50% to 70% by mass, based on the total mass of the composition.

5. A composition according to claim 4, **characterized in that** the oily portion comprises palm olein and passiflora oil.

6. A composition according to any one of claims 1 to 5, **characterized in that** the saponifying agent is sodium hydroxide and is present in an amount ranging from 15% to 25% by mass, based on the total mass of the composition.

7. A cosmetic composition according to any one of claims 1 to 7, **characterized by** comprising an antioxidant agent in an amount ranging from 0.00% to 5.00% by mass, based on the total mass of the composition.

8. A cosmetic composition according to claim 7, **characterized in that** the antioxidant agent is vitamin E.

9. A cosmetic composition according to any one of claims 1 to 8, **characterized by** being a toilet soap in paste.

10. A process for preparing a cosmetic composition, **characterized by** comprising the following steps:
a) preparing an oily portion comprising at least one plant oil comprising:
- a least 70% of fatty chain with 18 carbon atoms, C18, such chain may be either saturated or unsaturated;
- up to 20% of fatty chain with 16 carbon atoms, C16, such chain may be either saturated or unsaturated;
b) heating the oily portion of step a) up to a temperature of about 90°C to 95°C;
c) after heating, adding slowly at least one saponifying agent, under stirring and at high rotation;
d) adding slowly an oily portion;
e) checking the reduction of steam formed with the reaction.

11. A process according to claim 10, **characterized in that** in step (a) one adds an antioxidant agent, preferably vitamin E, to the oily portion.

12. Use of the cosmetic composition as defined in any one of claims 1 to 9, **characterized in that** it is for cleaning and hygiene of the skin.

13. Use of a cosmetic composition as defined in any one of claims 1 to 9, **characterized in that** it is for cleaning and hygiene of the skin.
